(19) **Europäisches Patentamt / European Patent Office / Office européen des brevets**

(11) **EP 4 434 462 B1**

(12) **EUROPEAN PATENT SPECIFICATION**

(45) Date of publication and mention
of the grant of the patent:
**09.07.2025 Bulletin 2025/28**

(51) International Patent Classification (IPC):
*A61B 5/318* (2021.01)    *A61B 5/349* (2021.01)
*A61B 5/00* (2006.01)    *G16H 50/30* (2018.01)
*G16H 50/70* (2018.01)    *G06N 3/02* (2006.01)

(21) Application number: **23217587.7**

(22) Date of filing: **18.12.2023**

(52) Cooperative Patent Classification (CPC):
**A61B 5/7264; A61B 5/318; A61B 5/349;
A61B 5/7267; G06N 3/045; G16H 50/20;
G16H 50/30; G16H 50/70;** G06N 3/02

(54) **METHOD AND SYSTEM FOR DETERMINING CARDIAC ABNORMALITIES USING CHAOS-BASED CLASSIFICATION MODEL FROM MULTI-LEAD ECG**

VERFAHREN UND SYSTEM ZUR BESTIMMUNG VON HERZANOMALIEN UNTER VERWENDUNG EINES CHAOSBASIERTEN KLASSIFIKATIONSMODELLS AUS EINEM MEHRELEKTRODEN-EKG

PROCÉDÉ ET SYSTÈME DE DÉTERMINATION D'ANOMALIES CARDIAQUES À L'AIDE D'UN MODÈLE DE CLASSIFICATION À BASE DE CHAOS À PARTIR D'ECG À DÉRIVATIONS MULTIPLES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **21.03.2023 IN 202321019383**

(43) Date of publication of application:
**25.09.2024 Bulletin 2024/39**

(73) Proprietor: **Tata Consultancy Services Limited
Maharashtra (IN)**

(72) Inventors:
• **SHARMA, Varsha**
  **700160 Kolkata, West Bengal (IN)**
• **GHOSE, Avik**
  **700160 Kolkata, West Bengal (IN)**
• **KHANDELWAL, Sundeep**
  **201309 Noida, Uttar Pradesh (IN)**
• **BHATTACHARYA, Sakyajit**
  **700160 Kolkata, West Bengal (IN)**

(74) Representative: **Goddar, Heinz J.
Boehmert & Boehmert
Anwaltspartnerschaft mbB
Pettenkoferstrasse 22
80336 München (DE)**

(56) References cited:
**WO-A1-2023/019022**

• **ELTRASS AHMED S ET AL: "Automated ECG multi-class classification system based on combining deep learning features with HRV and ECG measures", NEURAL COMPUTING AND APPLICATIONS, SPRINGER LONDON, LONDON, vol. 34, no. 11, 29 January 2022 (2022-01-29), pages 8755 - 8775, XP037828328, ISSN: 0941-0643, [retrieved on 20220129], DOI: 10.1007/ S00521-022-06889-Z**
• **XIAOZHE WANG ET AL: "Characteristic-Based Clustering for Time Series Data", DATA MINING AND KNOWLEDGE DISCOVERY, KLUWER ACADEMIC PUBLISHERS, BO, vol. 13, no. 3, 16 May 2006 (2006-05-16), pages 335 - 364, XP019434079, ISSN: 1573-756X, DOI: 10.1007/ S10618-005-0039-X**
• **IGNACIO PAUL SAMUEL ET AL: "Classification of Single-Lead Electrocardiograms: TDA Informed Machine Learning", 2019 18TH IEEE INTERNATIONAL CONFERENCE ON MACHINE LEARNING AND APPLICATIONS (ICMLA), IEEE, 16 December 2019 (2019-12-16), pages 1241 - 1246, XP033719681, DOI: 10.1109/ ICMLA.2019.00204**

**Description**

CROSS-REFERENCE TO RELATED APPLICATIONS AND PRIORITY

The present application claims priority to Indian application no. 202321019383, filed on March 21, 2023.

TECHNICAL FIELD

The embodiments herein generally relate to the field of Machine learning based cardiac abnormality detection and, more particularly, to a method and system for determining cardiac abnormalities using chaos-based classification model from multi-lead electrocardiogram (ECG) signals.

BACKGROUND

Machine Learning (ML) has a significant role in the automation of early diagnosis of diseases. Chaos theory provides a good non-linear dynamics model for a time-series. As well-known in the literature, biological systems like the human heart are non-linear but not completely random (stochastic). Hence, a normal electrocardiogram (ECG) signal may be best described as a signal having non-linear deterministic chaos. Studies have shown that there is a strong indication of ECG being a non-linear chaotic signal. Further, chaos theory parameters are used to extract pure ECG signals from noisy ECG. Attempts have been made towards usage of chaos-based features for classifying ECG into various classes of arrhythmia.

However, the features considered by the works in the literature still have limitations in accurately detecting whether the arrhythmia so noticed is progressing towards an associated disease or is an abrupt/random event due to the subject's current state. Further, these methods rely on windows of around 10 seconds of ECG recordings (time series data) to detect the presence of arrhythmia or cardiac abnormalities. Therefore, significant window periods pose a challenge in accurate AF Burden computation. Furthermore, the number of heartbeats is also high for a more substantial window duration; therefore, the chances of error in AF Burden computation are also high.

Thus, improvement in the accuracy of disease diagnosis associated with cardiac abnormalities is an open research area.

ELTRASS AHMED S ET AL: "Automated ECG multi-class classification system based on combining deep learning features with HRV and ECG measures" describes a hybrid approach of deep neural network combined with linear and nonlinear features extracted from Electrocardiogram (ECG) and heart rate variability (HRV) for ECG multi-class classification. The system enhances the ECG diagnosis performance by combining optimized deep learning features with an effective aggregation of ECG features and HRV measures using chaos theory and fragmentation analysis. Further, the constant-Q non-stationary Gabor transform technique is employed to convert the 1-D ECG signal into 2-D image which is sent to a pre-trained convolutional neural network structure, called AlexNet. The pair-wise feature proximity algorithm is employed to select the optimal features from the AlexNet output feature vector to be concatenated with the ECG and HRV measures. The concatenated features are sent to different types of classifiers to distinguish three distinct subjects, namely congestive heart failure, arrhythmia, and normal sinus rhythm (*Abstract*).

SUMMARY

Embodiments of the present disclosure present technological improvements as solutions to one or more of the above-mentioned technical problems recognized by the inventors in conventional systems. The invention is set out in appended set of claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1A is a functional block diagram of a system for determining cardiac abnormalities using a chaos-based classification model from multi-lead electrocardiogram (ECG) signals, in accordance with some embodiments of the present disclosure.
FIG. 1B illustrates an architectural overview of the system of FIG. 1A, in accordance with some embodiments of the present disclosure.

FIGS. 2A and 2B (collectively referred as FIG. 2) is a flow diagram illustrating a method for determining cardiac abnormalities using the chaos-based classification model from multi-lead ECG signals, using the system of FIG. 1A, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Improvement in accuracy of diseases diagnosis associated with cardiac abnormalities is an open research area. Appropriate feature selection to capture the underlying signs of a disease is critical in Machine Learning (ML) based approaches.

Embodiments of the present disclosure provide a method and system for determining cardiac abnormalities using chaos-based classification model from multi-lead electrocardiogram (ECG) signals. The method disclosed combines the commonly used chaos parameter with other set of chaos-related statistical parameters like non-linearity, self-similarity, Chebyshev distance and spectral flatness for a holistic approach towards the study of cardiac abnormalities. The method disclosed thus attempts to use above Machine Learning (ML) based measures for disease classification. The Chebyshev distance and the Spectral flatness have not been used so far to identify chaos in a disease detection environment.

Pathophysiology along with temporal information across leads is captured by the set of chaos-related features used herein, which contribute to improving the accuracy of detection of various cardiac diseases arising due to cardiac abnormalities such as Atrial Fibrillation (AF), Ventricular Fibrillation (VF), Sinus Arrhythmia, Ventricular Tachycardia (VT), complex conditions like VF followed by VT, and the like.

Furthermore, the method provides computation of percentage AF burden (AFB). The improved accuracy in detection of AF, effectively contributes to improved accuracy in percentage AFB.

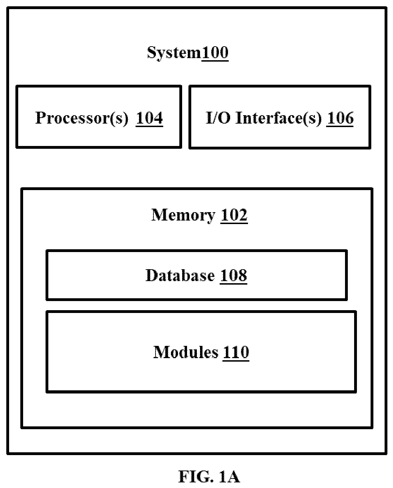

Referring now to the drawings, and more particularly to FIGS. 1A through 2B, where similar reference characters denote corresponding features consistently throughout the figures, there are shown preferred embodiments and these embodiments are described in the context of the following exemplary system and/or method.

FIG. 1A is a functional block diagram of a system 100 for determining cardiac abnormalities using a chaos-based classification model from multi-lead electrocardiogram (ECG) signals, in accordance with some embodiments of the present disclosure. In an embodiment, the system 100 includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like.

The I/O interface(s) 106 can include a variety of software and hardware interfaces, for example, a web interface, a graphical user interface to display subject's cardiac status and findings, and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to a number of external devices or to another server or devices.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

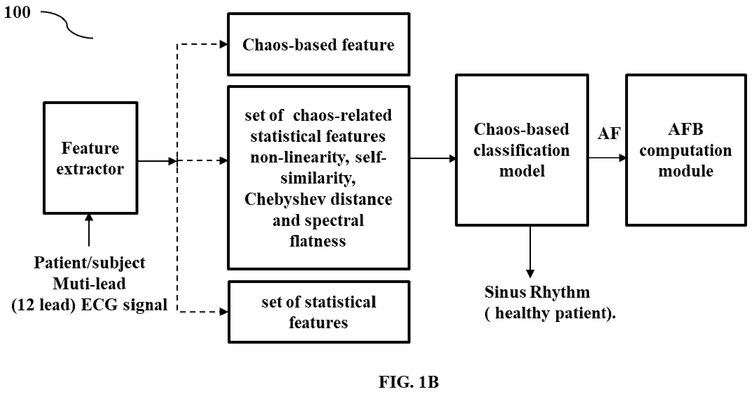

In an embodiment, the memory 102 includes a plurality of modules 110 such as a chaos-based classification model, a AFB computation module, and so on as depicted in FIG. 1B. The plurality of modules 110 include programs or coded instructions that supplement applications or functions performed by the system 100 for executing different steps involved in the process of determining cardiac abnormalities using the chaos-based classification model from multi-lead ECG signals, being performed by the system 100. The plurality of modules 110, amongst other things, can include routines, programs, objects, components, and data structures, which performs particular tasks or implement particular abstract data types. The plurality of modules 110 may also be used as, signal processor(s), node machine(s), logic circuitries, and/or any other device or component that manipulates signals based on operational instructions. Further, the plurality of modules 110 can be used by hardware, by computer-readable instructions executed by the one or more hardware processors 104, or by a combination thereof. The plurality of modules 110 can include various sub-modules (not shown).

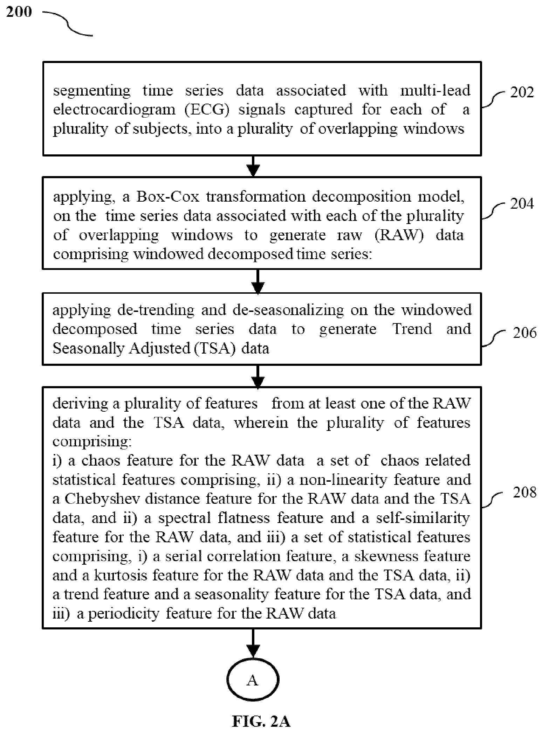

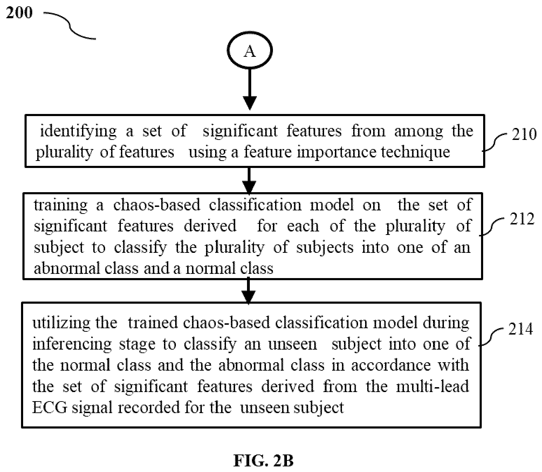

Further, the memory 102 may comprise information pertaining to input(s)/output(s) of each step performed by the processor(s) 104 of the system 100 and methods of the present disclosure. Further, the memory 102 includes a database 108. The database (or repository) 108 may include a plurality of abstracted piece of code for refinement and data that is processed, received, or generated as a result of the execution of the plurality of modules in the module(s) 110. Although the database 108 is shown internal to the system 100, it will be noted that, in alternate embodiments, the database 108 can also be implemented external to the system 100, and communicatively coupled to the system 100. The data contained within such external database may be periodically updated. For example, new data may be added into the database (not shown in FIG. 1A) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to FIG. 1B and steps in flow diagrams in FIG. 2.

FIG. 1B illustrates an architectural overview of the system of FIG. 1A, in accordance with some embodiments of the present disclosure. In one embodiment, wherein system 100 is built for AF detection, the system 100 performs i) data acquisition from the multi-lead ECG signals and ii) data analysis using the feature extraction module, the chaos-based classification module, and the AF burden computation module, explained in conjunction with FIG. 2. Further, data presentation is performed such that the data associated with results of chaos-based classification model and the AF burden is presented in a physician friendly manner. A Lorenz plot well known in the art can be used to display the findings such as report arrhythmia via the I/O interface 106 to a collocated or remotely located physician in a manner that physicians understand. The ECG recording analysis is detailed in steps of FIG. 2. It is to be noted, however, that the ECG analysis is lightweight and in health monitoring environments where a remote physician is not involved, the warning signal derived from findings can be provided to the patient/subject with an indication to visit their cardiologist. This is typically useful when the system 100 is implemented in the form of a smart wearable. However, for more clinical use-cases like implantable loop recorders, the system 100 can send the data to a cloud server, with minimal local processing to determine irregularity, for further analysis of Atrial Fibrillation (AF) and determination of the AF burden. In one embodiment, as this system 100 uses window-based time-series analysis methods, it is capable of providing AF burden values, which is considered an important factor. As detailed in studies and discussed in literature, AF burden is important factor to decide the treatment, and for AF ablation therapy.

FIGS. 2A and 2B (collectively referred as FIG. 2) is a flow diagram illustrating a method 200 for determining cardiac abnormalities using the chaos-based classification model from multi-lead ECG signals, using the system of FIG. 1A, in accordance with some embodiments of the present disclosure. In an embodiment, the system 100 comprises one or more data storage devices or the memory 102 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the system 100 as depicted in FIG. 1A and 1B and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

Referring to the steps of the method 200, at step 202 of the method 200, the one or more hardware processors 104 segment time series data associated with the multi-lead electrocardiogram (ECG) signals captured for each of a plurality of subjects, into a plurality of overlapping windows. For example, either of Atrial Fibrillation, Ventricular Fibrillation and Sinus Arrhythmia is selected as the abnormal class (Class 1) and Sinus Rhythm as the normal class (Class 2). From each class (normal and abnormal) ECG recording, also referred to as or ECG data, of 125 patients, is taken. Each ECG data (ECG recording) is a time series where the sampling rate is 500Hz. The segmenting is performed at time series window of 3 seconds (sec) (1500 observations) with 50% overlap. Unlike using a 10-sec window, as in most state-of-the-art approaches, the method utilizes a 3-sec window. For a case where the heart rate is low, at least 2-3 beats will be present in a 3-sec window. The error in identifying ectopic beat is less in a short period, which increases the accuracy in computing AF Burden. Additionally, analyzing the time series data over such a short period provides better information extraction for deriving more accurate insights as the number of windows is higher and increases the training instances. The information extraction from such a small window size is enabled due to the unique combination of the plurality of features used by method 200, as described in step 208 below.

At step 204 of the method 200, the one or more hardware processors 104 decomposes, using a Box-Cox transformation decomposition model, the time series data associated with each of the plurality of overlapping windows to generate raw (RAW) data comprising windowed decomposed time series.

At step 206 of the method 200, the one or more hardware processors 104 apply de-trending and de-seasonalizing on the windowed decomposed time series data to generate Trend and Seasonally Adjusted (TSA) data.

At step 208 of the method 200, the one or more hardware processors 104 derive the plurality of features, via the feature extraction module (FIG. 1B) executed by the one or more hardware processor 104, from at least one of the RAW data and the TSA data. In time series analysis, decomposing the data is necessary so that the data is fit for statistical measuring. Therefore, to obtain a precise and comprehensive calibration, some measures are calculated on both the raw time series data (referring as 'RAW' data), as well as the remaining time series after de-trending and de-seasonalizing (referring as "Trend and Seasonally Adjusted (TSA)" data). However, not all features can be computed on the TSA data because, after de-seasonalizing there is no such concept as periodicity. Thus, some features are both for RAW and TSA data, some features are for only RAW data and some only for TSA data.

The plurality of features comprise:

1) Chaos feature for the RAW data providing a uni-dimensional measure of cardiac abnormalities present in each windowed decomposed time series;

2) A set of chaos-related statistical features comprising, i) a non-linearity feature and a Chebyshev distance feature for the RAW data and the TSA data, and ii) a spectral flatness feature and a self-similarity feature for the RAW data, to add multiple dimensions to the chaos feature for generating a holistic view of the cardiac abnormalities.

3) A set of statistical features comprising, i) a serial correlation feature, a skewness feature and a kurtosis feature for the RAW data and the TSA data, ii) a trend feature and a seasonality feature for the TSA data, and iii) a periodicity feature for the RAW data, providing statistical distribution of the cardiac abnormalities.

In totality, there are 16 features as depicted in Table 1 below. Trend and seasonality only for TSA data, serial correlation, non-linearity, skewness, kurtosis, and Chebyshev distance for both data, self-similarity, chaos, periodicity, and spectral flatness only for RAW data.

Table 1

| Features | RAW Data | TSA Data |
|---|---|---|
| Trend | | ✓ |
| Seasonality | | ✓ |
| Serial Correlation | ✓ | ✓ |
| Non-Linearity | ✓ | ✓ |
| Skewness | ✓ | ✓ |
| Kurtosis | ✓ | ✓ |
| Chebyshev distance | ✓ | ✓ |
| Self-similarity | ✓ | |
| Chaos | ✓ | |
| Periodicity | ✓ | |
| Spectral flatness | ✓ | |

A trend pattern exists when there is a long-term change in the mean level, and seasonality of a time series is defined as a pattern that repeats itself over fixed intervals of time. Trend can be found using spline regression, and seasonality can be found using large partial autocorrelation at the seasonal lags. Periodicity determines the cyclic length of the time-series. Autocorrelation, skewness, and kurtosis are common features of time series that can be found using Box-Pierce statistics and method of moments, respectively.

Non-linearity, which determines structure of a time series, can be found using Teraesvirta Test. Self-similarity is basically the long-range dependence structure in a time series that can be found using Hurst exponent. Chaos is characterized by sensitive dependence on initial values. Recognizing and quantifying chaos in time series represent helps to understand the nature of random behavior and reveal the extent to which short-term forecasts may be improved. Chaos is found using Lyupanov Exponent. Chebyshev distance measures distance between two points as the maximum difference over any of their axis values. So, if there is P-P, R-R, T-T distances, of the ECG signal, Chebyshev distance will be the max, which means it captures if there is any of the above regularly missing, which is definitely a sign of abnormality. The spectral flatness is calculated by dividing the geometric mean of the power spectrum by the arithmetic mean of the power spectrum.

Among these features, chaos has been used before as an information theory parameter to identify heart-beat irregularities. However, only chaos cannot fully comprehend the extent of irregularities since it is a uni-dimensional measure. Hence other new features which have not been used so far to identify cardiac irregularities are appended by the method 200. The features, non-linearity, Chebyshev distance, spectral flatness, and self-similarity, are related with the properties of chaos and irregularity in a complex manner, and it is observed that only when they are taken together the holistic view towards ECG abnormalities is obtained.

In addition, some well-known time-series measures like autocorrelation, trend etc., are taken because they can give a detailed view of the statistical distribution.

Referring back to the steps of method 200, upon extracting the plurality of features, at step 210 of the method 200, the one or more hardware processors 104 identify a set of significant features from among the plurality of features using a feature importance technique. The method 200 in one example uses a 5-fold cross validation and run a random forest and a Gaussian SVM. Results are averaged over 5-folds. The Table 2 below shows average accuracy, sensitivity, specificity, and F1-score along with their standard deviations in parentheses. Here sensitivity is regarding the detection of abnormal cases and specificity is regarding the detection of normal cases. It was observed that random forest gives better result.

Table 2

|  | Accuracy | Sensitivity | Specificity | F1Score |
|---|---|---|---|---|
| SVM | 0.62(0.11) | 0.53(0.07) | 0.73(0.11) | 0.58(0.09) |
| RF | 0.73(0.06) | 0.79(0.03) | 0.71 0.05) | 0.75(0.06) |

**Feature importance:** Based on the random forest result, the feature importance analysis is performed using any feature importance model. Few example techniques are mentioned below:

1. MRMR (Maximum Relevance Minimum Redundancy): It ranks the most correlated feature with the target and the least correlated between themselves (feature set).
2. Information Gain: It calculates the reduction in entropy from the transformation of a dataset. It evaluates the information gain of each feature in context of the target variable.

In one example, it is observed that autocorrelation on the RAW data and periodicity are two most important features, while spectral flatness and kurtosis on the RAW data are the two least important features. However, with more experiments and new models being added, relative importance changes.

At step 212 of the method 200, the one or more hardware processors 104 train the chaos-based classification model on the set of significant features derived for each of the plurality of subject to classify the plurality of subjects into one of an abnormal class and a normal class. The classification of time series is based on their structural characteristics. Unlike other alternatives, the method 200 does not classify point values using a distance metric, rather it classifies based on global features extracted from the time series. The feature measures are obtained from each individual series and can be fed into arbitrary classification algorithms, including Support Vector Machine (SVM), random forest, naive Bayes, or neural network. Global measures describing the time series are obtained by applying statistical operations that best capture the underlying characteristics: trend, seasonality, periodicity, serial correlation, skewness, kurtosis, chaos, non-linearity, and self-similarity. Since the method 200uses extracted global measures, it reduces the dimensionality of the time series and is much less sensitive to missing or noisy data.

At step 214 of the method 200, the one or more hardware processors 104 utilize the trained chaos-based classification model during inferencing stage to classify an unseen subject into one of the normal class and the abnormal class. The set of significant features is derived from the multi-lead ECG signal recorded for the unseen subject for a predefined duration of an ECG recording, wherein the recorded ECG signal is segmented into the plurality of overlapping windows.

In one embodiment, wherein the chaos model is built and trained for AF detection, the abnormal class indicates the unseen subject suffering from Atrial Fibrillation (AF), and the normal class indicates the unseen subject to be healthy with Sinus Rhythm. Further, as depicted in FIG. 1B, the system 100 via the AFB computation module computes percentage of time spent in AF state by the subject within an observation period. For example, a patient is monitored for 24 hours, and time spent in AF is 2 hours, then percentage AF Burden is 8.33 (2/24*100). Obtaining accurate time spent in AF is dependent on accurate Normal/Abnormal classification. AF burden is usually computed over 14 days, however, for experiment herein, the system 100 calculates it over the time available due to the limitation of dataset length. For each subject, we calculate AFB % with respect to the total time monitored using below equation:

Computation of the AF burden (AFB%) for the unseen subject is as below:

$$D_{AF} = (N_{AF} \times (AF_{Avg} - D_{Ovlp})) + D_{ovlp},$$

$$D_{Ovlp} = AF_{Avg} \times 0.5, \text{ and}$$

$$AFB \% = D_{AF}/D_T \times 100, \qquad (1)$$

wherein, $D_{AF}$ is the duration of AF and $D_T$ is the predefined duration of the ECG recording, $N_{AF}$ is the number of AF windows detected for the unseen subject during the predefined duration of the ECG recording, $AF_{Avg}$ is an average of an AF time over the plurality of overlapping windows of the entire ECG recording, and $D_{Ovlp}$ is the AF time in the plurality of overlapping windows of the ECG recording, which is 50% of the $AF_{Avg}$.

**Significance of features in detecting the cardiac abnormalities and Extraction of features:** A uni-variate time series is the simplest form of temporal data and is a sequence of real numbers collected regularly in time, where each number represents a value. The time series herein is represented as an ordered set of n real-valued variables. Time series can be described using a variety of qualitative terms such as seasonal, trending, noisy, non-linear, chaos, etc. There are nine classical and advanced statistical features describing a time series' global characteristics. They are trend, seasonality, periodicity, serial correlation, skewness, kurtosis, non-linearity, self-similarity, and chaos. This collection of measures is quantified descriptors and can help provide a rich portrait of the nature of a time series. The features of trend, seasonality, periodic, serial correlation, skewness, and kurtosis have been widely used as exemplary measures in many time series feature-based research. Some advanced features are derived from the research on relatively new phenomena, which include non-linearity structure, self-similarity, and chaos. As a result, unique set of time series characteristics features are extracted by the method 200 as measures. The feature extraction process can also be considered as a dimensionality reduction procedure in time series data mining. Extracting the summarized characteristics of the time series can provide a more meaningful dimensionality reduction compared to other existing methods. By applying a statistical treatment to the analysis of time series data, datasets with long-length or different-length time series are pre-processed to produce a limited number of measures and are less sensitive to noise. These features concisely represent the relevant characteristics of each time series as a finite set of inputs to a clustering algorithm that can then discern similarities and differences between the time series. The outcome of feature extraction is a set of measures that can be fed into any clustering techniques of choice.

In time series analysis, decomposition is a critical step to transform the series into a format for statistical measuring. Therefore, to obtain a precise and comprehensive calibration, some measures are calculated on both the raw time series data (referring as 'RAW' data), as well as the remaining time series after de-trending and de-seasonalizing (referring as "Trend and Seasonally Adjusted (TSA)" data). But some features can only be calculated on raw data to obtain meaningful measures, such as periodicity, etc. As exhibited in the table 1, a total of thirteen measures are extracted from each time series including seven on the RAW data and six on the TSA data. These measures later become inputs to the chaos-based classification model. The thirteen measures are a finite set used to quantify the global characteristics of any time series, regardless of its length and missing values. For each of the features described below, a most appropriate way to measure the presence of the feature is used, and ultimately normalize the metric to [0, 1] to indicate the degree of presence of the feature. A measure near 0 for a certain time series indicates an absence of the feature, while a measure near 1 indicates a strong presence of the feature. The calculation of the measures and scaling transformations has been coded using the R language.

**Spectral flatness:** The spectral flatness or tonality coefficient is also known as 'Wiener Entropy' and is used to characterize the purity of an audio spectrum with respect to its tone. A high spectral flatness indicates a more white-noise signal. As well known in the art, spectral flatness is calculated by dividing the geometric mean of the power spectrum by the arithmetic mean of the power spectrum. Since spectral flatness gives the tonality of a signal, it should map to sinus rhythm of the ECG signal. In other words, high spectral flatness in ECG would indicate a wider distribution of the ECG spectrum at a given time. Therefore, arrhythmia should correlate well with the feature.

**Chebyshev distance:** Chebyshev distance is a metric defined on a vector space where the distance between two vectors is the greatest of their differences along any coordinate dimension. Thus, Chebyshev distance provides the maximum separation between two vectors. If an attempt is made to find the corresponding Chebyshev distance between two cardiac cycles, it will be along the dimension where the spectrum is maximum. If the distance is high, it means there is some anomaly in beat-to-beat dynamics indicating chaos, which can be a good indicator of arrhythmia.

**Chaos:** Many systems in nature that were previously considered random processes are now categorized as chaotic systems. Nonlinear dynamical systems often exhibit chaos, which is characterized by sensitive dependence on initial values, or more precisely by a positive Lyapunov Exponent (LE). Recognizing and quantifying chaos in time series represents important steps toward understanding the nature of random behavior and revealing the extent to which short-term forecasts may be improved. LE as a measure of the divergence of nearby trajectories has been used to qualifying chaos by giving a quantitative value. For a one-dimensional discrete time, series, an existing method demonstrated by Hilborn (1994) is used to calculate LE of a one-dimensional time series (RAW data).

**Non-linearity:** Nonlinear time series models have been used extensively in recent years to model complex dynamics not adequately represented use linear models. Because of the special characteristic (behavior) of time series data, the traditional linear models cannot handle the forecasting well compared to non-linear models. Therefore, non-linearity is an important characteristic of time series data to determine the selection of appropriate forecasting method. Herein, Teraesvirta's neural network test for time series data¨ non-linearity characteristics identification and extraction. It is a test for neglected nonlinearity likely to have power against a range of alternatives based on neural network model (augmented single-hidden-layer feed forward neural network model). The test is based on a test function chosen as the activation of 'phantom' hidden units. This measure is taken because non-linearity essentially means the underlying state space models need to be reconstructed time and again and possibly after certain periodic cycles. That means the heartbeat may show irregularity since they do not come from a unified state space model, which can be an indicator of arrhythmia.

**Self-similarity:** Processes with long-range dependence have attracted a good deal of attention from probabilistic and theoretical physicists. The subject of self-similarity and the estimation of statistical parameters of time series in the presence of long-range dependence are becoming more common in several fields of science, to which the time series analysis and forecasting on a recent research topic of network traffic, has drawn a particular attention. With such increasing importance of the 'self-similarity (long-range dependence)' as one of time series characteristics, this feature is included, although it is not widely used or is neglected in time series feature identification. The definition of self-similarity most related to the properties of time series is the self-similarity parameter Hurst exponent (H). The Self-similarity feature is only detected from the RAW data. This measure is taken because the less self-similar an ECG signal is, the less it is invariant in smaller parts. That means, the ECG signal may behave in microlevel quite differently than in macro level, e.g., a signal observed over few seconds vs a signal observed over 5 minutes. This leads to the logical conclusion that something happens in the signal that causes to deflect in long range from its usual cyclic pattern. This can be an indicator of heart bit irregularities and thus, arrhythmia.

**Trend and seasonality:** Trend and seasonality are common features of time series, and it is natural to characterize a time series by its degree of trend and seasonality. In addition, once the trend and seasonality of a time series have been measured, de-trend and de-seasonalize the time series can be done to enable additional features such as noise or chaos to be more easily detectable. A trend pattern exists when there is a long-term change in the mean level. To estimate the trend, a smooth nonparametric method, such as the penalized regression spline can be used. A seasonal pattern exists when a time series is influenced by seasonal factors, such as month of the year or day of the week. The seasonality of a time series is defined as a pattern that repeats itself over fixed intervals of time. In general, the seasonality can be found by identifying a large autocorrelation coefficient or a large partial autocorrelation coefficient at the seasonal lag. In an example implementation herein, the basic decomposition model using Box-Cox transformation is used.

**Periodicity:** Since the periodicity is very important for determining the seasonality and examining the cyclic pattern of the time series, the periodicity feature extraction becomes a necessity. Unfortunately, many time series available from the dataset in different domains do not always come with known frequency or regular periodicity. The method 200 discloses a new approach to measure the periodicity in univariate time series. The periodicity detection is only applied for RAW data. The time series is detrended using a regression spline with 3 knots and autocorrelations for all lags up to 1/3 of series length is determined. Thereafter peaks and troughs are identified in autocorrelation function. Frequency is the first peak provided with the following conditions:

- There is also a trough before it;
- The difference between peak and trough is at least 0.1; * the peak corresponds to positive correlation; and
- If no such peak is found, frequency is set to 1 (equivalent to non-seasonal).

**Serial Correlation:** A measure is extracted, which shows the degree of serial correlation of the dataset, to detect the series if it can fit a white noise model. The larger the degree is, noisier the series is. Normally in the white noise series, there are no recurring cycles (periodicity) in the data because each observation is completely independent of all other observations. A Box-Pierce statistics in used to estimate the serial correlation measure, and to extract the measures from both RAW and TSA data.

**Skewness and kurtosis:** Derivation is obvious, using well known method of moments. Using the above-mentioned set of features for estimating abnormality in cardiac rhythm has a strong pathophysiological basis. As we know the "sinus rhythm" present in ECG is a quasi-stationary signal, with the "quasi" factor contributing to heart-rate variability (HRV). However, there is a method in the madness and the HRV parameters have clinically sound limits within which they operate. Hence, using chaos as a measure of cardiac abnormality is actually checking the degree of entropy or disorderliness in the time-series. This may be due to some abnormality of the electrophysiology of the heart. For example, in Atrial Fibrillation (AF), the R-R peak intervals become chaotic, and the P-waves also are randomly missing, with intermittent F-waves (flutter) being present. This indicates a strong presence of chaos or randomness in the ECG signal, which is exploited by the method 200 for analysis. Hence, such analysis can be easily made explainable clinically.

The method and system disclosed herein provide a framework which uses chaos-based time series model on a multi-lead ECG signal to determine spatial distribution ( across subjects) of anomaly-related parameters in the ECG signal, and also depicts how such chaos-related components have a pathophysiological basis based on the underlying condition. The unique combination of 16 features used herein enables classifying ECG into various conditions and provides a robust and explainable approach to classify arrhythmia on the basis of ECG signals.

In future, such chaos-based features can be used to classify rare cardiac conditions accurately for which ample data is not available. This should be possible because the underlying method is not heavily empirical but uses very principled features based on knowledge of cardiac disorders.

The written description describes the subject matter herein to enable any person skilled in the art to make and use the embodiments. The scope of the subject matter embodiments is defined by the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. The words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

**1.** A processor implemented method (200) for determining cardiac abnormalities, the processor implemented method (200) comprising:

segmenting (202) via one or more hardware processors (104), time series data associated with multi-lead electrocardiogram, ECG, signals captured for each of a plurality of subjects, into a plurality of overlapping windows, wherein each of the plurality of overlapping windows has a window period of 3 seconds;

decomposing (204) via the one or more hardware processors (104), the time series data associated with each of the plurality of overlapping windows to generate raw time series data, RAW data, comprising windowed decomposed time series, wherein a basic decomposition model using a Box-Cox transformation model is used for generating the RAW data;

applying (206) via the one or more hardware processors (104), de-trending and de-seasonalizing on the windowed decomposed time series data to generate Trend and Seasonally Adjusted, TSA, data;

deriving (208) via the one or more hardware processors (104), a plurality of features from at least one of the RAW data and the TSA data, wherein the plurality of features comprising:

a chaos feature for the RAW data providing a uni-dimensional measure of the cardiac abnormalities present in each windowed decomposed time series;

a set of chaos-related statistical features comprising, i) a non-linearity feature and a Chebyshev distance feature for the RAW data and the TSA data, and ii) a spectral flatness feature and a self-similarity feature for the RAW data, to add multiple dimensions to the chaos feature for generating a holistic view of the cardiac abnormalities; and

a set of statistical features comprising, i) a serial correlation feature, a skewness feature and a kurtosis feature for the RAW data and the TSA data, ii) a trend feature and a seasonality feature for the TSA data, and iii) a periodicity feature for the RAW data, providing statistical distribution of the cardiac abnormalities;

identifying (210) via the one or more hardware processors (104), a set of significant features from among the plurality of features using a feature importance technique;

training (212) via the one or more hardware processors (104), a chaos-based classification model on the set of significant features derived for each of the plurality of subjects to classify the plurality of subjects into one of an abnormal class and a normal class; and

determining, via the one or more hardware processors (104), a cardiac abnormality utilizing the trained chaos-based classification model during an inferencing stage for an unseen subject, wherein the trained chaos-based classification model classifies the unseen subject into one of the normal class and the abnormal class in accordance with the set of significant features derived from the multi-lead ECG signal recorded for the unseen subject for a predefined duration of an ECG recording, the trained chaos-based classification being done by segmenting the ECG recording into the plurality of overlapping windows, wherein the abnormal class indicates the unseen subject suffering from the cardiac abnormality including one of Atrial Fibrillation, Ventricular Fibrillation, and Sinus Arrhythmia, and wherein the normal class indicates the unseen subject to be healthy with Sinus Rhythm.

2. The processor implemented method (200) as claimed in claim 1, comprising computing percentage AF burden (AFB%) for the unseen subject, wherein

$$\text{AFB \%} = D_{AF}/D_{T} \times 100,$$

$$D_{AF} = (N_{AF} \times (AF_{Avg} - D_{Ovlp})) + D_{ovlp},$$

$$D_{Ovlp} = AF_{Avg} \times 0.5,$$

and wherein, $D_{AF}$ is duration of the AF, $D_{T}$ is a predefined duration of the ECG recording, $N_{AF}$ is the number of AF windows detected for the unseen subject during the predefined duration of the ECG recording, $AF_{Avg}$ is an average of an AF time over the plurality of overlapping windows of the ECG recording, and $D_{Ovlp}$ is the AF time in the plurality of overlapping windows of the ECG recording, which is 50% of the $AF_{Avg}$.

3. The processor implemented method (200) as claimed in claim 1, wherein the chaos-based classification model is trained on the set of significant features derived for each of the plurality of subjects to classify the plurality of subjects into one of the abnormal class indicating Ventricular Fibrillation, VF, and the normal class indicating Sinus Rhythm.

4. The processor implemented method (200) as claimed in claim 1, wherein the chaos-based classification model is trained on the set of significant features derived for each of the plurality of subjects to classify the plurality of subjects into one of the abnormal class indicating Sinus Arrhythmia and the normal class indicating Sinus Rhythm.

5. A system (100) for determining cardiac abnormalities, the system (100) comprising:

a memory (102) storing instructions;
one or more Input/Output, I/O, interfaces (106); and
one or more hardware processors (104) coupled to the memory (102) via the one or more I/O interfaces (106), wherein the one or more hardware processors (104) are configured by the instructions to:

segment time series data associated with multi-lead electrocardiogram, ECG, signals captured for each of a plurality of subjects, into a plurality of overlapping windows, wherein each of the plurality of overlapping windows has a window period of 3 seconds;
decompose the time series data associated with each of the plurality of overlapping windows to generate raw time series data, RAW data, comprising windowed decomposed time series, wherein a basic decomposition model using a Box-Cox transformation model is used to generate the RAW data;
apply de-trending and de-seasonalizing on the windowed decomposed time series data to generate Trend and Seasonally Adjusted, TSA, data;
derive a plurality of features from at least one of the RAW data and the TSA data, wherein the plurality of features comprising:

a chaos feature for the RAW data providing a uni-dimensional measure of cardiac abnormalities present in each windowed decomposed time series;
a set of chaos-related statistical features comprising, i) a non-linearity feature and a Chebyshev distance feature for the RAW data and the TSA data, and ii) a spectral flatness feature and a self-similarity feature for the RAW data, to add multiple dimensions to the chaos feature for generating a holistic view of the cardiac abnormalities; and
a set of statistical features comprising, i) a serial correlation feature, a skewness feature and a kurtosis feature for the RAW data and the TSA data, ii) a trend feature and a seasonality feature for the TSA data, and iii) a periodicity feature for the RAW data, providing statistical distribution of the cardiac abnormalities;

identify a set of significant features from among the plurality of features using a feature importance technique;
train a chaos-based classification model on the set of significant features derived for each of the plurality of subjects to classify the plurality of subjects into one of an abnormal class and a normal class; and
determine a cardiac abnormality utilizing the trained chaos-based classification model during an inferencing stage for an unseen subject, wherein the trained chaos-based classification model classifies the unseen subject into one of the normal class and the abnormal class in accordance with the set of significant features derived from the multi-lead ECG signal recorded for the unseen subject for a predefined duration of an ECG recording, the trained chaos-based classification being done by segmenting the ECG recording into the plurality of overlapping windows, wherein the abnormal class indicates the unseen subject suffering from the cardiac abnormality including one of Atrial Fibrillation, Ventricular Fibrillation, and Sinus Arrhythmia, and wherein the normal class indicates the unseen subject to be healthy with Sinus Rhythm.

6. The system (100) as claimed in claim 5, wherein the one or more hardware processors (104) are configured to compute percentage AF burden, AFB%, for the unseen subject, wherein

$$\text{AFB \%} = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0.5,$$

and wherein, $D_{AF}$ is duration of the AF, $D_T$ is a predefined duration of the ECG recording, $N_{AF}$ is the number of AF windows detected for the unseen subject during the predefined duration of the ECG recording, $AF_{Avg}$ is an average of an AF time over the plurality of overlapping windows of the ECG recording, and $D_{Ovlp}$ is the AF time in the plurality of overlapping windows of the ECG recording, which is 50% of the $AF_{Avg}$.

7. The system (100) as claimed in claim 5, wherein the chaos-based classification model is trained on the set of significant features derived for each of the plurality of subjects to classify the plurality of subjects into one of the abnormal class indicating Ventricular Fibrillation, VF, and the normal class indicating Sinus Rhythm.

8. The system (100) as claimed in claim 5, wherein the chaos-based classification model is trained on the set of significant features derived for each of the plurality of subjects to classify the plurality of subjects into one of the abnormal class indicating Sinus Arrhythmia and the normal class indicating Sinus Rhythm.

9. One or more non-transitory machine-readable information storage mediums (102) comprising one or more instructions which when executed by one or more hardware processors (104) cause:

segmenting time series data associated with multi-lead electrocardiogram, ECG, signals captured for each of a plurality of subjects, into a plurality of overlapping windows, wherein each of the plurality of overlapping windows has a window period of 3 seconds;
decomposing the time series data associated with each of the plurality of overlapping windows to generate raw time series data, RAW data, comprising windowed decomposed time series, wherein a basic decomposition model using a Box-Cox transformation model is used for generating the RAW data;
applying de-trending and de-seasonalizing on the windowed decomposed time series data to generate Trend and Seasonally Adjusted, TSA, data;
deriving a plurality of features from at least one of the RAW data and the TSA data, wherein the plurality of features comprising:

a chaos feature for the RAW data providing a uni-dimensional measure of the cardiac abnormalities present in each windowed decomposed time series;
a set of chaos-related statistical features comprising, i) a non-linearity feature and a Chebyshev distance feature for the RAW data and the TSA data, and ii) a spectral flatness feature and a self-similarity feature for the RAW data, to add multiple dimensions to the chaos feature for generating a holistic view of the cardiac abnormalities; and
a set of statistical features comprising, i) a serial correlation feature, a skewness feature and a kurtosis feature for the RAW data and the TSA data, ii) a trend feature and a seasonality feature for the TSA data, and iii) a periodicity feature for the RAW data, providing statistical distribution of the cardiac abnormalities;

identifying a set of significant features from among the plurality of features using a feature importance technique;
training a chaos-based classification model on the set of significant features derived for each of the plurality of subjects to classify the plurality of subjects into one of an abnormal class and a normal class; and
determining a cardiac abnormality utilizing the trained chaos-based classification model during an inferencing stage for an unseen subject, wherein the trained chaos-based classification model classifies the unseen subject into one of the normal class and the abnormal class in accordance with the set of significant features derived from the multi-lead ECG signal recorded for the unseen subject for a predefined duration of an ECG recording, the trained chaos-based classification being done by segmenting the ECG recording into the plurality of overlapping windows, wherein the abnormal class indicates the unseen subject suffering from the cardiac abnormality including one of Atrial Fibrillation, Ventricular Fibrillation, and Sinus Arrhythmia, and wherein the normal class indicates the unseen subject to be healthy with Sinus Rhythm.

10. The one or more non-transitory machine-readable information storage mediums (102) of claim 9, further comprising instructions for computing percentage AF burden, AFB%, for the unseen subject, wherein

$$AFB \% = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0.5,$$

and wherein, $D_{AF}$ is duration of the AF, $D_T$ is a predefined duration of the ECG recording, $N_{AF}$ is the number of AF windows detected for the unseen subject during the predefined duration of the ECG recording, $AF_{Avg}$ is an average of an AF time over the plurality of overlapping windows of the ECG recording, and $D_{Ovlp}$ is the AF time in the plurality of overlapping windows of the ECG recording, which is 50% of the $AF_{Avg}$.

## Patentansprüche

**1.** Prozessorimplementiertes Verfahren (200) zum Bestimmen von Herzanomalien, wobei das prozessorimplementierte Verfahren (200) Folgendes umfasst:

Segmentieren (202), über einen oder mehrere Hardwareprozessoren (104), von Zeitreihendaten, die mit Mehrleitungs-Elektrokardiogramm-, EKG-, Signalen assoziiert sind, die für jedes einer Mehrzahl von Subjekten erfasst wurden, in eine Mehrzahl von überlappenden Fenstern, wobei jedes der Mehrzahl von überlappenden Fenstern eine Fensterperiode von 3 Sekunden aufweist;

Zerlegen (204), über den einen oder die mehreren Hardwareprozessoren (104), der Zeitreihendaten, die mit jedem der Mehrzahl von überlappenden Fenstern assoziiert sind, um rohe Zeitreihendaten, RAW-Daten, zu erzeugen, die gefensterte zerlegte Zeitreihen umfassen, wobei ein Basiszerlegungsmodell, das ein Box-Cox-Transformationsmodell verwendet, zum Erzeugen der RAW-Daten verwendet wird;

Anwenden (206), über den einen oder die mehreren Hardwareprozessoren (104), von Detrending und Desaisonalisierung auf die gefensterten zerlegten Zeitreihendaten, um Trend- und saisonal angepasste, TSA-, Daten zu erzeugen;

Ableiten (208), über den einen oder die mehreren Hardwareprozessoren (104), einer Mehrzahl von Merkmalen aus mindestens einem der RAW-Daten und der TSA-Daten, wobei die Mehrzahl von Merkmalen Folgendes umfasst:

ein Chaos-Merkmal für die RAW-Daten, das ein eindimensionales Maß der Herzanomalien bereitstellt, die in jeder gefensterten zerlegten Zeitreihe vorhanden sind;

einen Satz von chaosbezogenen statistischen Merkmalen, umfassend i) ein Nichtlinearitätsmerkmal und ein Tschebyschjew-Abstandsmerkmal für die RAW-Daten und die TSA-Daten und ii) ein spektrales Flachheitsmerkmal und ein Selbstähnlichkeitsmerkmal für die RAW-Daten, um dem Chaos-Merkmal mehrere Dimensionen hinzuzufügen, um eine holistische Ansicht der Herzanomalien zu erzeugen; und

einen Satz von statistischen Merkmalen, umfassend i) ein serielles Korrelationsmerkmal, ein Schiefemerkmal und ein Kurtosis-Merkmal für die RAW-Daten und die TSA-Daten, ii) ein Trendmerkmal und ein Saisonalitätsmerkmal für die TSA-Daten und iii) ein Periodizitätsmerkmal für die RAW-Daten, das eine statistische Verteilung der Herzanomalien bereitstellt;

Identifizieren (210), über den einen oder die mehreren Hardwareprozessoren (104), eines Satzes von signifikanten Merkmalen aus der Mehrzahl von Merkmalen unter Verwendung einer Merkmalswichtigkeitstechnik;

Trainieren (212), über den einen oder die mehreren Hardwareprozessoren (104), eines chaosbasierten Klassifizierungsmodells an dem Satz von signifikanten Merkmalen, die für jedes der Mehrzahl von Subjekten abgeleitet wurden, um die Mehrzahl von Subjekten in eine von einer anormalen Klasse und einer normalen Klasse zu klassifizieren; und

Bestimmen, über den einen oder die mehreren Hardwareprozessoren (104), einer Herzanomalie unter Verwendung des trainierten chaosbasierten Klassifizierungsmodells während einer Inferenzphase für ein ungesehenes Subjekt, wobei das trainierte chaosbasierte Klassifizierungsmodell das ungesehene Subjekt in eine von der normalen Klasse und der anormalen Klasse gemäß dem Satz von signifikanten Merkmalen klassifiziert, die von dem Mehrleitungs-EKG-Signal abgeleitet wurden, das für das ungesehene Subjekt für eine vordefinierte Dauer einer EKG-Aufzeichnung aufgezeichnet wurde, wobei die trainierte chaosbasierte Klassifizierung durch Segmentieren der EKG-Aufzeichnung in die Mehrzahl von überlappenden Fenstern erfolgt, wobei die anormale Klasse das ungesehene Subjekt angibt, das an der Herzanomalie leidet, einschließlich eines von Vorhofflimmern, Kammerflimmern und Sinusarrhythmie, und wobei die normale Klasse das ungesehene Subjekt angibt, mit Sinusrhythmus gesund zu sein.

**2.** Prozessorimplementiertes Verfahren (200) nach Anspruch 1, umfassend das Berechnen der prozentualen AF-Belastung (AFB%) für das ungesehene Subjekt, wobei

$$AFB\% = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0{,}5,$$

und wobei $D_{AF}$ die Dauer der AF ist, $D_T$ eine vordefinierte Dauer der EKG-Aufzeichnung ist, $N_{AF}$ die Anzahl von AF-Fenstern ist, die für das ungesehene Subjekt während der vordefinierten Dauer der EKG-Aufzeichnung erfasst wurden, $AF_{Avg}$ ein Durchschnitt einer AF-Zeit über die Mehrzahl von überlappenden Fenstern der EKG-Aufzeichnung ist und $D_{Ovlp}$ die AF-Zeit in der Mehrzahl von überlappenden Fenstern der EKG-Aufzeichnung ist, die 50% der $AF_{Avg}$ beträgt.

3.  Prozessorimplementiertes Verfahren (200) nach Anspruch 1, wobei das chaosbasierte Klassifizierungsmodell an dem Satz von signifikanten Merkmalen trainiert wird, die für jedes der Mehrzahl von Subjekten abgeleitet wurden, um die Mehrzahl von Subjekten in eine von der anormalen Klasse, die Kammerflimmern, VF, angibt, und der normalen Klasse, die Sinusrhythmus angibt, zu klassifizieren.

4.  Prozessorimplementiertes Verfahren (200) nach Anspruch 1, wobei das chaosbasierte Klassifizierungsmodell an dem Satz von signifikanten Merkmalen trainiert wird, die für jedes der Mehrzahl von Subjekten abgeleitet wurden, um die Mehrzahl von Subjekten in eine von der anormalen Klasse, die Sinusarrhythmie angibt, und der normalen Klasse, die Sinusrhythmus angibt, zu klassifizieren.

5.  System (100) zum Bestimmen von Herzanomalien, wobei das System (100) Folgendes umfasst:

    einen Speicher (102), der Anweisungen speichert;
    eine oder mehrere Eingabe/Ausgabe-, E/A-, Schnittstellen (106); und
    einen oder mehrere Hardwareprozessoren (104), die mit dem Speicher (102) über die eine oder die mehreren E/A-Schnittstellen (106) gekoppelt sind, wobei der eine oder die mehreren Hardwareprozessoren (104) durch die Anweisungen konfiguriert sind zum:

    Segmentieren von Zeitreihendaten, die mit Mehrleitungs-Elektrokardiogramm-, EKG-, Signalen assoziiert sind, die für jedes einer Mehrzahl von Subjekten erfasst wurden, in eine Mehrzahl von überlappenden Fenstern, wobei jedes der Mehrzahl von überlappenden Fenstern eine Fensterperiode von 3 Sekunden aufweist;
    Zerlegen der Zeitreihendaten, die mit jedem der Mehrzahl von überlappenden Fenstern assoziiert sind, um rohe Zeitreihendaten, RAW-Daten, zu erzeugen, die gefensterte zerlegte Zeitreihen umfassen, wobei ein Basiszerlegungsmodell, das ein Box-Cox-Transformationsmodell verwendet, zum Erzeugen der RAW-Daten verwendet wird;
    Anwenden von Detrending und Desaisonalisierung auf die gefensterten zerlegten Zeitreihendaten, um Trend- und saisonal angepasste, TSA-, Daten zu erzeugen;
    Ableiten einer Mehrzahl von Merkmalen aus mindestens einem der RAW-Daten und der TSA-Daten, wobei die Mehrzahl von Merkmalen Folgendes umfasst:

    ein Chaos-Merkmal für die RAW-Daten, das ein eindimensionales Maß von Herzanomalien bereitstellt, die in jeder gefensterten zerlegten Zeitreihe vorhanden sind;
    einen Satz von chaosbezogenen statistischen Merkmalen, umfassend i) ein Nichtlinearitätsmerkmal und ein Tschebyschjew-Abstandsmerkmal für die RAW-Daten und die TSA-Daten und ii) ein spektrales Flachheitsmerkmal und ein Selbstähnlichkeitsmerkmal für die RAW-Daten, um dem Chaos-Merkmal mehrere Dimensionen hinzuzufügen, um eine holistische Ansicht der Herzanomalien zu erzeugen; und
    einen Satz von statistischen Merkmalen, umfassend i) ein serielles Korrelationsmerkmal, ein Schiefemerkmal und ein Kurtosis-Merkmal für die RAW-Daten und die TSA-Daten, ii) ein Trendmerkmal und ein Saisonalitätsmerkmal für die TSA-Daten und iii) ein Periodizitätsmerkmal für die RAW-Daten, das eine statistische Verteilung der Herzanomalien bereitstellt;

    Identifizieren eines Satzes von signifikanten Merkmalen aus der Mehrzahl von Merkmalen unter Verwendung einer Merkmalswichtigkeitstechnik;

Trainieren eines chaosbasierten Klassifizierungsmodells an dem Satz von signifikanten Merkmalen, die für jedes der Mehrzahl von Subjekten abgeleitet wurden, um die Mehrzahl von Subjekten in eine von einer anormalen Klasse und einer normalen Klasse zu klassifizieren; und

Bestimmen einer Herzanomalie unter Verwendung des trainierten chaosbasierten Klassifizierungsmodells während einer Inferenzphase für ein ungesehenes Subjekt, wobei das trainierte chaosbasierte Klassifizierungsmodell das ungesehene Subjekt in eine von der normalen Klasse und der anormalen Klasse gemäß dem Satz von signifikanten Merkmalen klassifiziert, die von dem Mehrleitungs-EKG-Signal abgeleitet wurden, das für das ungesehene Subjekt für eine vordefinierte Dauer einer EKG-Aufzeichnung aufgezeichnet wurde, wobei die trainierte chaosbasierte Klassifizierung durch Segmentieren der EKG-Aufzeichnung in die Mehrzahl von überlappenden Fenstern erfolgt, wobei die anormale Klasse das ungesehene Subjekt angibt, das an der Herzanomalie leidet, einschließlich eines von Vorhofflimmern, Kammerflimmern und Sinusarrhythmie, und wobei die normale Klasse das ungesehene Subjekt angibt, mit Sinusrhythmus gesund zu sein.

6. System (100) nach Anspruch 5, wobei der eine oder die mehreren Hardwareprozessoren (104) konfiguriert sind, um die prozentuale AF-Belastung, AFB%, für das ungesehene Subjekt zu berechnen, wobei

$$AFB\% = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0{,}5,$$

und wobei $D_{AF}$ die Dauer der AF ist, $D_T$ eine vordefinierte Dauer der EKG-Aufzeichnung ist, $N_{AF}$ die Anzahl von AF-Fenstern ist, die für das ungesehene Subjekt während der vordefinierten Dauer der EKG-Aufzeichnung erfasst wurden, $AF_{Avg}$ ein Durchschnitt einer AF-Zeit über die Mehrzahl von überlappenden Fenstern der EKG-Aufzeichnung ist und $D_{Ovlp}$ die AF-Zeit in der Mehrzahl von überlappenden Fenstern der EKG-Aufzeichnung ist, die 50% der $AF_{Avg}$ beträgt.

7. System (100) nach Anspruch 5, wobei das chaosbasierte Klassifizierungsmodell an dem Satz von signifikanten Merkmalen trainiert wird, die für jedes der Mehrzahl von Subjekten abgeleitet wurden, um die Mehrzahl von Subjekten in eine von der anormalen Klasse, die Kammerflimmern, VF, angibt, und der normalen Klasse, die Sinusrhythmus angibt, zu klassifizieren.

8. System (100) nach Anspruch 5, wobei das chaosbasierte Klassifizierungsmodell an dem Satz von signifikanten Merkmalen trainiert wird, die für jedes der Mehrzahl von Subjekten abgeleitet wurden, um die Mehrzahl von Subjekten in eine von der anormalen Klasse, die Sinusarrhythmie angibt, und der normalen Klasse, die Sinusrhythmus angibt, zu klassifizieren.

9. Ein oder mehrere nichtflüchtige maschinenlesbare Informationsspeichermedien (102), die eine oder mehrere Anweisungen umfassen, die bei Ausführung durch einen oder mehrere Hardwareprozessoren (104) Folgendes bewirken:

Segmentieren von Zeitreihendaten, die mit Mehrleitungs-Elektrokardiogramm-, EKG-, Signalen assoziiert sind, die für jedes einer Mehrzahl von Subjekten erfasst wurden, in eine Mehrzahl von überlappenden Fenstern, wobei jedes der Mehrzahl von überlappenden Fenstern eine Fensterperiode von 3 Sekunden aufweist;

Zerlegen der Zeitreihendaten, die mit jedem der Mehrzahl von überlappenden Fenstern assoziiert sind, um rohe Zeitreihendaten, RAW-Daten, zu erzeugen, die gefensterte zerlegte Zeitreihen umfassen, wobei ein Basiszerlegungsmodell, das ein Box-Cox-Transformationsmodell verwendet, zum Erzeugen der RAW-Daten verwendet wird;

Anwenden von Detrending und Desaisonalisierung auf die gefensterten zerlegten Zeitreihendaten, um Trend- und saisonal angepasste, TSA-, Daten zu erzeugen;

Ableiten einer Mehrzahl von Merkmalen aus mindestens einem der RAW-Daten und der TSA-Daten, wobei die Mehrzahl von Merkmalen Folgendes umfasst:

ein Chaos-Merkmal für die RAW-Daten, das ein eindimensionales Maß der Herzanomalien bereitstellt, die in jeder gefensterten zerlegten Zeitreihe vorhanden sind;

einen Satz von chaosbezogenen statistischen Merkmalen, umfassend i) ein Nichtlinearitätsmerkmal und ein Tschebyschjew-Abstandsmerkmal für die RAW-Daten und die TSA-Daten und ii) ein spektrales Flachheitsmerkmal und ein Selbstähnlichkeitsmerkmal für die RAW-Daten, um dem Chaos-Merkmal mehrere Dimensionen hinzuzufügen, um eine holistische Ansicht der Herzanomalien zu erzeugen; und

einen Satz von statistischen Merkmalen, umfassend i) ein serielles Korrelationsmerkmal, ein Schiefemerkmal und ein Kurtosis-Merkmal für die RAW-Daten und die TSA-Daten, ii) ein Trendmerkmal und ein Saisonalitätsmerkmal für die TSA-Daten und iii) ein Periodizitätsmerkmal für die RAW-Daten, das eine statistische Verteilung der Herzanomalien bereitstellt;

Identifizieren eines Satzes von signifikanten Merkmalen aus der Mehrzahl von Merkmalen unter Verwendung einer Merkmalswichtigkeitstechnik;

Trainieren eines chaosbasierten Klassifizierungsmodells an dem Satz von signifikanten Merkmalen, die für jedes der Mehrzahl von Subjekten abgeleitet wurden, um die Mehrzahl von Subjekten in eine von einer anormalen Klasse und einer normalen Klasse zu klassifizieren; und

Bestimmen einer Herzanomalie unter Verwendung des trainierten chaosbasierten Klassifizierungsmodells während einer Inferenzphase für ein ungesehenes Subjekt, wobei das trainierte chaosbasierte Klassifizierungsmodell das ungesehene Subjekt in eine von der normalen Klasse und der anormalen Klasse gemäß dem Satz von signifikanten Merkmalen klassifiziert, die von dem Mehrleitungs-EKG-Signal abgeleitet wurden, das für das ungesehene Subjekt für eine vordefinierte Dauer einer EKG-Aufzeichnung aufgezeichnet wurde, wobei die trainierte chaosbasierte Klassifizierung durch Segmentieren der EKG-Aufzeichnung in die Mehrzahl von überlappenden Fenstern erfolgt, wobei die anormale Klasse das ungesehene Subjekt angibt, das an der Herzanomalie leidet, einschließlich eines von Vorhofflimmern, Kammerflimmern und Sinusarrhythmie, und wobei die normale Klasse das ungesehene Subjekt angibt, mit Sinusrhythmus gesund zu sein.

10. Ein oder mehrere nichtflüchtige maschinenlesbare Informationsspeichermedien (102) nach Anspruch 9, ferner umfassend Anweisungen zum Berechnen der prozentualen AF-Belastung, AFB%, für das ungesehene Subjekt, wobei

$$AFB\% = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0{,}5,$$

und wobei $D_{AF}$ die Dauer der AF ist, $D_T$ eine vordefinierte Dauer der EKG-Aufzeichnung ist, $N_{AF}$ die Anzahl von AF-Fenstern ist, die für das ungesehene Subjekt während der vordefinierten Dauer der EKG-Aufzeichnung erfasst wurden, $AF_{Avg}$ ein Durchschnitt einer AF-Zeit über die Mehrzahl von überlappenden Fenstern der EKG-Aufzeichnung ist und $D_{Ovlp}$ die AF-Zeit in der Mehrzahl von überlappenden Fenstern der EKG-Aufzeichnung ist, die 50% der $AF_{Avg}$ beträgt.

## Revendications

1. Procédé mis en œuvre par processeur (200) pour déterminer des anomalies cardiaques, le procédé mis en œuvre par processeur (200) comprenant :

la segmentation (202), par l'intermédiaire d'un ou plusieurs processeurs matériels (104), de données de série temporelle associées à des signaux d'électrocardiogramme, ECG, à dérivations multiples capturés pour chacun d'une pluralité de sujets, en une pluralité de fenêtres se chevauchant, dans lequel chacune de la pluralité de fenêtres se chevauchant a une période de fenêtre de 3 secondes ;

la décomposition (204), par l'intermédiaire des un ou plusieurs processeurs matériels (104), des données de série temporelle associées à chacune de la pluralité de fenêtres se chevauchant pour générer des données de série temporelle brutes, RAW, comprenant des séries temporelles décomposées en fenêtres, dans lequel un modèle de décomposition de base utilisant un modèle de transformation de Box-Cox est utilisé pour générer les données RAW ;

l'application (206), par l'intermédiaire des un ou plusieurs processeurs matériels (104), d'une détendance et d'une désaisonnalisation sur les données de série temporelle décomposées en fenêtres pour générer des données de tendance et d'ajustement saisonnier, TSA ;

la dérivation (208), par l'intermédiaire des un ou plusieurs processeurs matériels (104), d'une pluralité de caractéristiques à partir d'au moins l'une des données RAW et des données TSA, dans lequel la pluralité de caractéristiques comprend :

une caractéristique de chaos pour les données RAW fournissant une mesure unidimensionnelle des anomalies cardiaques présentes dans chaque série temporelle décomposée en fenêtres ;

un ensemble de caractéristiques statistiques liées au chaos comprenant, i) une caractéristique de non-linéarité et une caractéristique de distance de Tchebychev pour les données RAW et les données TSA, et ii) une caractéristique de planéité spectrale et une caractéristique d'auto-similarité pour les données RAW, pour ajouter de multiples dimensions à la caractéristique de chaos pour générer une vue holistique des anomalies cardiaques ; et

un ensemble de caractéristiques statistiques comprenant, i) une caractéristique de corrélation sérielle, une caractéristique d'asymétrie et une caractéristique de kurtosis pour les données RAW et les données TSA, ii) une caractéristique de tendance et une caractéristique de saisonnalité pour les données TSA, et iii) une caractéristique de périodicité pour les données RAW, fournissant une distribution statistique des anomalies cardiaques ;

l'identification (210), par l'intermédiaire des un ou plusieurs processeurs matériels (104), d'un ensemble de caractéristiques significatives parmi la pluralité de caractéristiques en utilisant une technique d'importance de caractéristique ;

l'apprentissage (212), par l'intermédiaire des un ou plusieurs processeurs matériels (104), d'un modèle de classification basé sur le chaos sur l'ensemble de caractéristiques significatives dérivées pour chacun de la pluralité de sujets pour classer la pluralité de sujets dans l'une d'une classe anormale et d'une classe normale ; et

la détermination, par l'intermédiaire des un ou plusieurs processeurs matériels (104), d'une anomalie cardiaque en utilisant le modèle de classification basé sur le chaos appris pendant une étape d'inférence pour un sujet non vu, dans lequel le modèle de classification basé sur le chaos appris classe le sujet non vu dans l'une de la classe normale et de la classe anormale conformément à l'ensemble de caractéristiques significatives dérivées du signal ECG multiconducteur enregistré pour le sujet non vu pendant une durée prédéfinie d'un enregistrement ECG, la classification basée sur le chaos appris étant effectuée en segmentant l'enregistrement ECG dans la pluralité de fenêtres se chevauchant, dans lequel la classe anormale indique le sujet non vu souffrant de l'anomalie cardiaque incluant l'une d'une fibrillation auriculaire, d'une fibrillation ventriculaire et d'une arythmie sinusale, et dans lequel la classe normale indique le sujet non vu comme étant en bonne santé avec un rythme sinusal.

2. Procédé mis en œuvre par processeur (200) selon la revendication 1, comprenant le calcul d'un pourcentage de charge AF (AFB%) pour le sujet non vu, dans lequel

$$AFB \% = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0{,}5,$$

et dans lequel, $D_{AF}$ est la durée de l'AF, $D_T$ est une durée prédéfinie de l'enregistrement ECG, $N_{AF}$ est le nombre de fenêtres AF détectées pour le sujet non vu pendant la durée prédéfinie de l'enregistrement ECG, $AF_{Avg}$ est une moyenne d'un temps AF sur la pluralité de fenêtres se chevauchant de l'enregistrement ECG, et $D_{Ovlp}$ est le temps AF dans la pluralité de fenêtres se chevauchant de l'enregistrement ECG, qui est 50 % de l'$AF_{Avg}$.

3. Procédé mis en œuvre par processeur (200) selon la revendication 1, dans lequel le modèle de classification basé sur le chaos est appris sur l'ensemble de caractéristiques significatives dérivées pour chacun de la pluralité de sujets pour classer la pluralité de sujets dans l'une de la classe anormale indiquant une fibrillation ventriculaire, VF, et de la classe normale indiquant un rythme sinusal.

**4.** Procédé mis en œuvre par processeur (200) selon la revendication 1, dans lequel le modèle de classification basé sur le chaos est appris sur l'ensemble de caractéristiques significatives dérivées pour chacun de la pluralité de sujets pour classer la pluralité de sujets dans l'une de la classe anormale indiquant une arythmie sinusale et de la classe normale indiquant un rythme sinusal.

**5.** Système (100) pour déterminer des anomalies cardiaques, le système (100) comprenant :

une mémoire (102) stockant des instructions ;
une ou plusieurs interfaces d'entrée/sortie, E/S, (106) ; et
un ou plusieurs processeurs matériels (104) couplés à la mémoire (102) par l'intermédiaire des une ou plusieurs interfaces E/S (106), dans lequel les un ou plusieurs processeurs matériels (104) sont configurés par les instructions pour :

segmenter des données de série temporelle associées à des signaux d'électrocardiogramme, ECG, à dérivations multiples capturés pour chacun d'une pluralité de sujets, en une pluralité de fenêtres se chevauchant, dans lequel chacune de la pluralité de fenêtres se chevauchant a une période de fenêtre de 3 secondes ;
décomposer les données de série temporelle associées à chacune de la pluralité de fenêtres se chevauchant pour générer des données de série temporelle brutes, RAW, comprenant des séries temporelles décomposées en fenêtres, dans lequel un modèle de décomposition de base utilisant un modèle de transformation de Box-Cox est utilisé pour générer les données RAW ;
appliquer une détendance et une désaisonnalisation sur les données de série temporelle décomposées en fenêtres pour générer des données de tendance et d'ajustement saisonnier, TSA ;
dériver une pluralité de caractéristiques à partir d'au moins l'une des données RAW et des données TSA, dans lequel la pluralité de caractéristiques comprend :

une caractéristique de chaos pour les données RAW fournissant une mesure unidimensionnelle des anomalies cardiaques présentes dans chaque série temporelle décomposée en fenêtres ;
un ensemble de caractéristiques statistiques liées au chaos comprenant, i) une caractéristique de non-linéarité et une caractéristique de distance de Tchebychev pour les données RAW et les données TSA, et ii) une caractéristique de planéité spectrale et une caractéristique d'auto-similarité pour les données RAW, pour ajouter de multiples dimensions à la caractéristique de chaos pour générer une vue holistique des anomalies cardiaques ; et
un ensemble de caractéristiques statistiques comprenant, i) une caractéristique de corrélation sérielle, une caractéristique d'asymétrie et une caractéristique de kurtosis pour les données RAW et les données TSA, ii) une caractéristique de tendance et une caractéristique de saisonnalité pour les données TSA, et iii) une caractéristique de périodicité pour les données RAW, fournissant une distribution statistique des anomalies cardiaques ;

identifier un ensemble de caractéristiques significatives parmi la pluralité de caractéristiques en utilisant une technique d'importance de caractéristique ;
apprendre un modèle de classification basé sur le chaos sur l'ensemble de caractéristiques significatives dérivées pour chacun de la pluralité de sujets pour classer la pluralité de sujets dans l'une d'une classe anormale et d'une classe normale ; et
déterminer une anomalie cardiaque en utilisant le modèle de classification basé sur le chaos appris pendant une étape d'inférence pour un sujet non vu, dans lequel le modèle de classification basé sur le chaos appris classe le sujet non vu dans l'une de la classe normale et de la classe anormale conformément à l'ensemble de caractéristiques significatives dérivées du signal ECG multiconducteur enregistré pour le sujet non vu pendant une durée prédéfinie d'un enregistrement ECG, la classification basée sur le chaos appris étant effectuée en segmentant l'enregistrement ECG dans la pluralité de fenêtres se chevauchant, dans lequel la classe anormale indique le sujet non vu souffrant de l'anomalie cardiaque incluant l'une d'une fibrillation auriculaire, d'une fibrillation ventriculaire et d'une arythmie sinusale, et dans lequel la classe normale indique le sujet non vu comme étant en bonne santé avec un rythme sinusal.

**6.** Système (100) selon la revendication 5, dans lequel les un ou plusieurs processeurs matériels (104) sont configurés pour calculer un pourcentage de charge AF, AFB%, pour le sujet non vu, dans lequel

$$AFB \% = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0,5,$$

et dans lequel, $D_{AF}$ est la durée de l'AF, $D_T$ est une durée prédéfinie de l'enregistrement ECG, $N_{AF}$ est le nombre de fenêtres AF détectées pour le sujet non vu pendant la durée prédéfinie de l'enregistrement ECG, $AF_{Avg}$ est une moyenne d'un temps AF sur la pluralité de fenêtres se chevauchant de l'enregistrement ECG, et $D_{Ovlp}$ est le temps AF dans la pluralité de fenêtres se chevauchant de l'enregistrement ECG, qui est 50 % de l'$AF_{Avg}$.

7. Système (100) selon la revendication 5, dans lequel le modèle de classification basé sur le chaos est appris sur l'ensemble de caractéristiques significatives dérivées pour chacun de la pluralité de sujets pour classer la pluralité de sujets dans l'une de la classe anormale indiquant une fibrillation ventriculaire, VF, et de la classe normale indiquant un rythme sinusal.

8. Système (100) selon la revendication 5, dans lequel le modèle de classification basé sur le chaos est appris sur l'ensemble de caractéristiques significatives dérivées pour chacun de la pluralité de sujets pour classer la pluralité de sujets dans l'une de la classe anormale indiquant une arythmie sinusale et de la classe normale indiquant un rythme sinusal.

9. Un ou plusieurs supports de stockage d'informations lisibles par machine non transitoires (102) comprenant une ou plusieurs instructions qui, lorsqu'elles sont exécutées par un ou plusieurs processeurs matériels (104), amènent :

la segmentation de données de série temporelle associées à des signaux d'électrocardiogramme, ECG, à dérivations multiples capturés pour chacun d'une pluralité de sujets, en une pluralité de fenêtres se chevauchant, dans lequel chacune de la pluralité de fenêtres se chevauchant a une période de fenêtre de 3 secondes ;
la décomposition des données de série temporelle associées à chacune de la pluralité de fenêtres se chevauchant pour générer des données de série temporelle brutes, RAW, comprenant des séries temporelles décomposées en fenêtres, dans lequel un modèle de décomposition de base utilisant un modèle de transformation de Box-Cox est utilisé pour générer les données RAW ;
l'application d'une détendance et d'une désaisonnalisation sur les données de série temporelle décomposées en fenêtres pour générer des données de tendance et d'ajustement saisonnier, TSA ;
la dérivation d'une pluralité de caractéristiques à partir d'au moins l'une des données RAW et des données TSA, dans lequel la pluralité de caractéristiques comprend :

une caractéristique de chaos pour les données RAW fournissant une mesure unidimensionnelle des anomalies cardiaques présentes dans chaque série temporelle décomposée en fenêtres ;
un ensemble de caractéristiques statistiques liées au chaos comprenant, i) une caractéristique de non-linéarité et une caractéristique de distance de Tchebychev pour les données RAW et les données TSA, et ii) une caractéristique de planéité spectrale et une caractéristique d'auto-similarité pour les données RAW, pour ajouter de multiples dimensions à la caractéristique de chaos pour générer une vue holistique des anomalies cardiaques ; et
un ensemble de caractéristiques statistiques comprenant, i) une caractéristique de corrélation sérielle, une caractéristique d'asymétrie et une caractéristique de kurtosis pour les données RAW et les données TSA, ii) une caractéristique de tendance et une caractéristique de saisonnalité pour les données TSA, et iii) une caractéristique de périodicité pour les données RAW, fournissant une distribution statistique des anomalies cardiaques ;

l'identification d'un ensemble de caractéristiques significatives parmi la pluralité de caractéristiques en utilisant une technique d'importance de caractéristique ;
l'apprentissage d'un modèle de classification basé sur le chaos sur l'ensemble de caractéristiques significatives dérivées pour chacun de la pluralité de sujets pour classer la pluralité de sujets dans l'une d'une classe anormale et d'une classe normale ; et
la détermination d'une anomalie cardiaque en utilisant le modèle de classification basé sur le chaos appris pendant une étape d'inférence pour un sujet non vu, dans lequel le modèle de classification basé sur le chaos appris classe le sujet non vu dans l'une de la classe normale et de la classe anormale conformément à l'ensemble de caractéristiques significatives dérivées du signal ECG multiconducteur enregistré pour le sujet non vu pendant une durée prédéfinie d'un enregistrement ECG, la classification basée sur le chaos appris étant effectuée en segmentant l'enregistrement ECG dans la pluralité de fenêtres se chevauchant, dans lequel la classe anormale indique le sujet non vu souffrant de l'anomalie cardiaque incluant l'une d'une fibrillation auriculaire, d'une fibrillation ventriculaire et d'une arythmie sinusale, et dans lequel la classe normale indique le sujet non vu comme étant en bonne santé avec un rythme sinusal.

10. Un ou plusieurs supports de stockage d'informations lisibles par machine non transitoires (102) selon la revendication 9, comprenant en outre des instructions pour calculer un pourcentage de charge AF, AFB%, pour le sujet non vu, dans lequel

$$AFB \% = D_{AF}/D_T \times 100,$$

$$D_{AF} = (N_{AF} \times (AF_{Avg} - D_{Ovlp})) + D_{ovlp},$$

$$D_{Ovlp} = AF_{Avg} \times 0,5,$$

et dans lequel, $D_{AF}$ est la durée de l'AF, $D_T$ est une durée prédéfinie de l'enregistrement ECG, $N_{AF}$ est le nombre de fenêtres AF détectées pour le sujet non vu pendant la durée prédéfinie de l'enregistrement ECG, $AF_{Avg}$ est une moyenne d'un temps AF sur la pluralité de fenêtres se chevauchant de l'enregistrement ECG, et $D_{Ovlp}$ est le temps AF dans la pluralité de fenêtres se chevauchant de l'enregistrement ECG, qui est 50 % de l'$AF_{Avg}$.

System<u>100</u>

Processor(s) <u>104</u>

I/O Interface(s) <u>106</u>

Memory <u>102</u>

Database <u>108</u>

Modules <u>110</u>

FIG. 1A

100

Patient/subject Muti-lead (12 lead) ECG signal → Feature extractor

Chaos-based feature

set of chaos-related statistical features non-linearity, self-similarity, Chebyshev distance and spectral flatness

set of statistical features

Chaos-based classification model

AF → AFB computation module

Sinus Rhythm (healthy patient).

FIG. 1B

200

segmenting time series data associated with multi-lead electrocardiogram (ECG) signals captured for each of a plurality of subjects, into a plurality of overlapping windows

202

applying, a Box-Cox transformation decomposition model, on the time series data associated with each of the plurality of overlapping windows to generate raw (RAW) data comprising windowed decomposed time series:

204

applying de-trending and de-seasonalizing on the windowed decomposed time series data to generate Trend and Seasonally Adjusted (TSA) data

206

deriving a plurality of features from at least one of the RAW data and the TSA data, wherein the plurality of features comprising:
i) a chaos feature for the RAW data a set of chaos related statistical features comprising, ii) a non-linearity feature and a Chebyshev distance feature for the RAW data and the TSA data, and ii) a spectral flatness feature and a self-similarity feature for the RAW data, and iii) a set of statistical features comprising, i) a serial correlation feature, a skewness feature and a kurtosis feature for the RAW data and the TSA data, ii) a trend feature and a seasonality feature for the TSA data, and iii) a periodicity feature for the RAW data

208

A

**FIG. 2A**

**200**

A

identifying a set of significant features from among the plurality of features using a feature importance technique ⟋ 210

training a chaos-based classification model on the set of significant features derived for each of the plurality of subject to classify the plurality of subjects into one of an abnormal class and a normal class ⟋ 212

utilizing the trained chaos-based classification model during inferencing stage to classify an unseen subject into one of the normal class and the abnormal class in accordance with the set of significant features derived from the multi-lead ECG signal recorded for the unseen subject ⟋ 214

**FIG. 2B**

EP 4 434 462 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321019383

**Non-patent literature cited in the description**

- **ELTRASS AHMED S et al.** *Automated ECG multi-class classification system based on combining deep learning features with HRV and ECG measures*

25